(19) Europäisches Patentamt
European Patent Office
Office européen des brevets

(11) Numéro de publication: **0 213 996**
**B1**

(12) # FASCICULE DE BREVET EUROPEEN

(45) Date de publication du fascicule du brevet: **06.09.89**

(51) Int. Cl.⁴: **C 08 F 20/60,** C 07 K 1/00

(21) Numéro de dépôt: **86401603.5**

(22) Date de dépôt: **17.07.86**

(54) **Polymères ramifiés à base de lipopeptides formant des cristaux liquides thermotropes, et lyotropes leurs applications, et monomères correspondants.**

(30) Priorité: **19.07.85 FR 8511121**

(43) Date de publication de la demande: **11.03.87 Bulletin 87/11**

(45) Mention de la délivrance du brevet: **06.09.89 Bulletin 89/36**

(84) Etats contractants désignés: **AT BE CH DE FR GB IT LI LU NL SE**

(56) Documents cités: **EP-A- 0 105 777**

(73) Titulaire: **CENTRE NATIONAL DE LA RECHERCHE SCIENTIFIQUE (CNRS), 15, Quai Anatole France, F-75007 Paris (FR)**

(72) Inventeur: **Gallot, Bernard, 220 rue Rodolphe Richard, F-45160 Olivet (FR)**
Inventeur: **Douy, André, 269 rue des Briandes, F-45160 Olivet (FR)**

(74) Mandataire: **Phélip, Bruno et al, c/o Cabinet Harié & Phélip 21, rue de La Rochefoucauld, F-75009 Paris (FR)**

**EP 0 213 996 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des polymères ramifiés à base de lipopeptides, qui sont dans un état mésomorphe à température ambiante, ainsi que leurs applications et les monomères qui servent à leur préparation.

Au sens de la présente description, on entend par «polymères ramifiés à base de lipopeptides», des polymères comportant des chaînes pendantes lipopeptidiques, polymères également connus sous l'expression «polymères en peigne».

La présente invention concerne aussi, lorsque les polymères ramifiés sont amphipatiques, leurs applications comme agents émulsifiants et moussants.

On a déjà décrit un certain nombre de polymères ayant des propriétés de cristaux liquides et résultant de la fixation de groupes mésogènes à la chaîne d'un polymère par l'intermédiaire d'un bras espaceur déformable. Ces polymères présentent les propriétés de cristaux liquides de faible masse moléculaire avec les qualités des polymères, et ont un grand intérêt technique.

On pourra se référer par exemple à l'ouvrage «Polymer liquid crystals» edité par A. CIFERRI, W.R. KRIGBAUM et R.B. MEYER et publié par Academic press en 1982; on peut citer, en particulier, tous les systèmes optoélectroniques d'affichage sur écrans ou encore les affichages sensibles à la température.

La présente invention concerne des polymères qui sont dans un état mésomorphe dès la température ambiante et présentent successivement deux types de phase: smectique, puis nématique, avant de donner une phase liquide isotrope à une température nettement plus élevée. Or, il est particulièrement souhaitable de n'avoir, pour les applications en affichage, que des mésophases qui soient stables dans un large domaine de température.

En outre, et ceci est tout à fait inattendu, ils sont à la fois thermotropes et lyotropes, c'est-à-dire que le changement de phase peut être observé, soit en élevant la température du polymère pur, soit par dilution de ces polymères dans certains solvants. Du fait de leur caractère lyotrope, ces composés sont très adaptés pour dissoudre des colorants variés, ioniques ou non, dont on connaît l'utilité en affichage. La plupart des cristaux liquides actuels sont décomposés par ces colorants ioniques sous l'action des champs électriques.

Les polymères selon l'invention résultent de la polymérisation d'un ou plusieurs monomères répondant à la formule (I):

$$H_2C=\underset{R}{\underset{|}{C}}-\underset{O}{\underset{\|}{C}}-Z-B-NH-(AA)_p \qquad (I)$$

formule dans laquelle:

— R représente H ou un groupe alkyle inférieur en $C_1$ à $C_3$;

— Z est O ou NH;

— B répond à la formule:

$$-(CH_2)_{q1}-Z_1-(CH_2)_{q2}$$

formule dans laquelle $q_1$ et $q_2$ sont des entiers supérieurs ou égaux à 1, dont la somme est inférieure à 32 et $Z_1$ représente une simple liaison ou O, S, NH, CO ou CO-NH;

— $(AA)_p$ représente un radical peptidique constitué de p alpha-amino-acides aliphatiques couplés, et fixé à B par sa fonction carboxyle, chaque amino-acide comportant moins de 15 atomes de carbone et étant éventuellement substitué sur un carbone de la chaîne par un groupe acide ou ester cabroxylique, amide ou amine, la fonction amine terminale du radical peptidique pouvant être acylée par un groupe R'CO, R' étant un radical alkyle renfermant de 1 à 4 atomes de carbone, ou par tout autre groupe stable dans les conditions de la synthèse; et

— p représentant un nombre compris entre 1 et 200.

B comporte notamment de 6 à 24 atomes de carbone.

Les mélanges de ces polymères et copolymères sont un autre objet de l'invention. Ils peuvent être constitués de composés ayant des degrés de polymérisation divers, mélanges résultant de la réaction de polymérisation du groupement vinylique; ils peuvent aussi être des mélanges dérivant de mélanges de monomères obtenus lors de la réaction de polymérisation des polypeptides terminaux, et ayant donc des p différents.

En outre, les copolymères résultants de la polymérisation statistique des monomères de formule (I) avec des composés de formule (II):

$$H_2C=\overset{R}{\overset{|}{C}}-\overset{O}{\overset{\|}{C}}-Z(CH_2)_n-R^o \qquad (II)$$

formule dans laquelle:

— n est compris entre 2 et 30;

— R et Z ont la même signification que ci-dessus; et

— $R^o$ représente $CH_3$ ou OH,

sont aussi un objet de l'invention.

Les amino-acides à l'origine de la partie terminale de la chaîne ramifiée du polymère, représentée par (AA)p, ont des chaînes latérales hydrophiles ou hydrophobes. Cette partie terminale résulte de la condensation, par liaison peptidique, des amino-acides entre eux et sur B, de telle sorte que si B est $(CH_2)_3$- et p = 1, -Z-B-NH-$(AA)_p$ signifie:

$$-HN-(CH_2)_3-NH-\underset{O}{\underset{\|}{C}}-\underset{NHR_2}{\underset{|}{CH}}-(CH_2)_r-Z_2$$

formule dans laquelle:

— r est un nombre entier compris entre 0 et 5;

— $Z_2$ représente un groupe amino, substitué ou non, ou un groupe acide carboxylique, ester ou amide; et

— $R_2$ représente H ou COR',

ou encore si p = 3, -Z-B-NH$(AA)_3$ signifie:

$-HN-(CH_2)_3-NH-C-CH-(CH_2)_r-Z_2$
$\quad\quad\quad\quad\quad\quad\| \quad |$
$\quad\quad\quad\quad\quad\quad O \; NH$
$\quad\quad\quad\quad\quad\quad\quad |$
$\quad\quad\quad\quad\quad\quad\quad C=O$
$\quad\quad\quad\quad\quad\quad\quad |$
$\quad\quad\quad\quad\quad\quad\quad CH-(CH_23)_r-Z_2$
$\quad\quad\quad\quad\quad\quad\quad |$
$\quad\quad\quad\quad\quad\quad\quad NH$
$\quad\quad\quad\quad\quad\quad\quad |$
$\quad\quad\quad\quad\quad\quad\quad C=O$
$\quad\quad\quad\quad\quad\quad\quad |$
$\quad\quad\quad\quad\quad\quad\quad CH-(CH_2)_r-Z_2$
$\quad\quad\quad\quad\quad\quad\quad |$
$\quad\quad\quad\quad\quad\quad\quad NHR_2$

formule dans laquelle:

— r et $Z_2$ ont les mêmes significations que ci-dessus; et

— $R_2$ représente H ou -COR', R' étant un reste alkyle en $C_1$ à $C_4$.

Les amino-acides préférés sont mentionnés dans le tableau I ci-après:

TABLEAU I

| Abréviation | Formule développée | Dénomination commune de l'aminoacide |
|---|---|---|
| Sar | $NH-CH_2-COOH$<br>\|<br>$CH_3$ | Sarcosine |
| Orn | $H_2N-CH-COOH$<br>\|<br>$(CH_2)_3-NH_2$ | Ornitine |
| K | $H_2N-CH-COOH$<br>\|<br>$(CH_2)_4-NH_2$ | Lysine |
| E | $H_2N-CH-COOH$<br>\|<br>$(CH_2)_2-COOH$ | Acide glutamique |
| D | $H_2N-CH-COOH$<br>\|<br>$CH_2-COOH$ | Acide aspartique |
| EnEt | $H_2N-CH-COOH$<br>\|<br>$(CH_2)_2-CO-NH-(CH_2)_2-OH$ | Hydroxyéthylglutamine |
| EnPro | $H_2N-CH-COOH$<br>\|<br>$(CH_2)_2-CO-NH-(CH_2)_3OH$ | Hydroxypropylglutamine |
| EnBu | $H_2N-CH-COOH$<br>\|<br>$(CH_2)_2-CO-NH-(CH_2)_4OH$ | Hydroxybutylglutamine |
| EnPen | $H_2N-CH-COOH$<br>\|<br>$(CH_2)_2-CO-NH-(CH_2)_5OH$ | Hydroxypentylglutamine |
| DnEt | $H_2N-CH-COOH$<br>\|<br>$CH_2-CO-NH-(CH_2)_2OH$ | Hydroxyéthylaspartamine |

TABLEAU 1 (suite)

| Abréviation | Formule développée | Dénomination commune de l'aminoacide |
|---|---|---|
| Ebzl | $H_2N-CH-COOH$<br>\|<br>$(CH_2)_2-COO-CH_2-C_6H_5$ | Glutamate de benzyle |
| Dbzl | $H_2N-CH-COOH$<br>\|<br>$CH_2-COO-CH_2-C_6H_5$ | Aspartate de benzyle |
| KTfa | $H_2N-CH-COOH$<br>\|<br>$(CH_2)_4-NH-CO-CF_3$ | Trifluoroacétyl-lysine |
| OrnTfa | $H_2N-CH-COOH$<br>\|<br>$(CH_2)_3-NH-CO-CF_3$ | Trifluoroacétyl-ornithine |
| KCbz | $H_2N-CH-COOH$<br>\|<br>$(CH_2)_4-NH-COO-CH_2-C_6H_5$ | Carbobenzoxy-lysine |
| OrnCbz | $H_2N-CH-COOH$<br>\|<br>$(CH_2)_3-NH-COO-CH_2-C_6H_5$ | Carbobenzoxy-ornithine |
| EMe | $H_2N-CH-COOH$<br>\|<br>$(CH_2)_2-COOCH_3$ | Glutamate de méthyle |
| EEt | $H_2N-CH-COOH$<br>\|<br>$(CH_2)_2COO-C_2H_5$ | Glutamate d'éthyle |
| Gly | $H_2N-CH_2-COOH$ | Glycine |

Les amino-acides à chaînes latérales hydprophobes sont, de préférence, utilisés sous forme de copolymères avec des monomères acrylates ou acrylamides, étant donné les difficultés de synthèse.

Pour les amino-acides à chaîne latérale hydrophile, on préfère tout particulièrement la sarcosine (SAR), la lysine, l'ornithine, l'acide glutamique, l'acide aspartique, l'hydroxyéthylglutamine (EnEt) et l'hydroxyéthylaspartamine (DnEt); parmi les aminoacides à chaîne latérale hydrophobe, on préfère la trifluoroacétyllysine (KTfa), la carbobenzoxylysine (KCbz), la trifluoroacétyl-ornithine, les glutamates d'alkyle et le glutamate de benzyle (Ebzl).

Il est aussi possible d'avoir, comme groupe terminal, un polypeptide résultant du couplage d'aminoacides différents; toutefois, il est préférable qu'ils soient identiques, ou au moins de structure proche, pour que l'hélice du polypeptide à chaîne latérale hydrophobe ne soit pas déstabilisée et la formation des cristaux liquides, gênée.

Pour les amino-acides à chaîne latérale hydrophile, on préfère $p < 60$, tandis que pour ceux à chaîne latérale hydrophobe, on préfère $p < 200$.

Les monomères de formule I peuvent être préparés suivant la nature de l'amino-acide et la valeur de p:

— a) soit en faisant préalablement réagir une molécule polymérisable:

$$\begin{array}{c} R \\ | \\ H_2C=C-COX, \end{array}$$

formule dans laquelle COX représente un dérivé réactif d'acide carboxylique, tel qu'un ester, chlorure d'acide, anhydride ou autres, sur un composé de formule $HZ-B-NH_2$ dans laquelle Z et B ont les significations indiquées ci-dessus, ou sur un de ses dérivés dans lesquels la fonction amine est temporairement bloquée pour obtenir un produit de formule:

$$\begin{array}{c} R \\ | \\ H_2C=C-CO-Z-B-NH_2 \end{array}$$

sur lequel on fixera le, ou les groupes dérivés d'acides aminés, en faisant réagir, soit le N-carboxyanhydride d'alpha-amino-acide, soit, dans les conditions des synthèses peptidiques, un alpha-amino-acide dont la fonction amine en alpha est protégée, par exemple, par le groupe t-butyloxycarbonyle; dans tous les cas, les autres groupes réactifs éventuels sont protégés de façon classique.

— b) soit en faisant réagir la molécule polymérisable:

R
|
$H_2C=C$-CO-X

où CO-X a la même signification qu'au paragraphe a) précédent sur la fonction amine d'un amino-acide à longue chaîne:

$$NH_2\text{-}(CH_2)_{n1}\text{-}COOH$$

avec n1 représentant un nombre entier de 2 à 11, puis en activant la fonction acide du dérivé obtenu sous forme d'ester de succinimidyle, enfin en faisant réagir l'ester activé avec une diamine:

$$HN_2\text{-}(CH_2)_{n2}\text{-}NH_2$$

avec n2 = entier = 2 à 20,
pour obtenir l'amine polymérisable:

$$CH_2=CR\text{-}CONH\text{-}(CH_2)_{n1}\text{-}CONH\text{-}(CH_2)_{n2}\text{-}NH_2$$

sur laquelle on peut fixer le ou les groupes dérivés d'acides aminés de la manière décrite en a).

— c) soit en faisant réagir sur le molécule polymérisable précédemment citée:

R
|
$H_2C=C$-COX,

un composé de formule HZ-B-(AA)p, dans laquelle Z, B, AA et p ont les mêmes significations que précédemment et pour lequel la fonction amine du dernier AA est bloquée, ou un mélange de ces composés qui ont Z, B, AA identiques mais dont les p diffèrent. Les revendications 17 à 19 décrivent des procédés préférés pour la préparation de monomères selon l'invention.

Les conditions de ces diverses réactions sont bien connues de l'homme de métier.

Dans le procédé selon a), une des fonctions amines de la diamine de formule $H_2N$-B-$NH_2$ ou la fonction amine de l'amino-alcool HO-B-$NH_2$ sera bloquée par un groupement protecteur, tel que les groupements t-butyloxycarbonyle, trifluoroacétyle, 2-nitrophénylsulfonyle ou 9-fluorénylméthyloxycarbonyle par des méthodes connues en elles-mêmes.

Dans l'un ou l'autre des procédés a) et b), on préfère appliquer la méthode au N-carboxyanhydride (NCA) pour les p supérieurs à 5 environ et pouvant aller jusqu'à 200; la méthode de couplage peptidique ne permet pratiquement d'obtenir que des p faibles, de 4 au plus et dans ce cas, on peut, soit faire réagir l'amino-acide à fixer, dont on a protégé la fonction amine, sur l'amine polymérisable, et répéter l'opération pour coupler plusieurs amino-acides, soit faire réagir directement le di- ou tripeptide dont la fonction amine terminale a été protégée.

Dans le procédé selon c), pour préparer le composé de formule HZ-B-(AA)p, on fait réagir le N-carboxyanhydride d'un amino-acide sur l'amine HZ-B-$NH_2$, après avoir protégé de façon classique ZH lorsque Z représente NH. On bloque ensuite la fonction amine libre du polypeptide fixé par exemple par action de R'COCl sur le produit obtenu en solution dans le THF et en présence de triéthylamine; on peut introduire d'autres groupes du moment qu'ils sont stables dans les étapes ultérieures de la synthèse et non volumineux. Cette méthode est particulièrement utile lorsque AA représente KTfa, OrnTfa, Ebzl et Dbzl. Les conditions pour obtenir HZ-B-$(AA)_p$ à partir du dérivé protégé diffèrent suivant la nature de AA; si AA est Sar, KTfa, OrnTfa, Ebzl ou Dbzl, on élimine le groupe protecteur BOC, par action de HCl anhydre en solution dans le THF; pour obtenir les composés où AA est K et Orn, on fait réagir ensuite de la pipéridine sur les dérivés où AA est KTfa et OrnTfa en solution dans le THF, ou encore on traite ces produits dérivés par NaOH en solution aqueuse puis méthanolique; si AA est Ebzl ou Dbzl, il est préférable de bloquer préalablement la fonction amine par un groupe 2-nitrophénylsulfonyle plutôt que BOC, qui pourra être éliminé après le couplage des amino-acides, en milieu acide faible; à partir de ces derniers composés, on pourra obtenir ceux dans lesquels AA est E et D par action d'un acide puis d'une base en milieu aqueux; enfin, dans le cas de Kbz et OrnCbz, on bloquera la fonction amine de préférence, par un groupe trifluoroacétyle ou 9-fluorénylméthyloxycarbonyle qui pourra être éliminé en milieu basique, sans hydrolyse des groupes Cbz. Dans le cas d'autres amino-acides porteurs de groupes «sensibles», l'homme du métier sera à même de déterminer quel groupe protecteur chosir pour la fonction amine, pour que son élimination soit sans action sur le polypeptide fixé.

La polymérisation du groupe acrylique est réalisée de façon classique, en solution aqueuse avec le persulfate de potassium comme amorceur pour les monomères solubles dans l'eau à pH neutre ou basique, ou en solution organique dans le THF ou le chloroforme, avec un peroxyde ou l'azobisisobutyronitrile comme amorceur; on peut copolymériser divers monomères de formule (I), ou un monomère de formule (I) avec un acrylate d'alkyle ou d'hydroxyalkyle de formule:

$H_2C=CCOOR_4$
|
$R_3$

ou un acrylamide de formule:

$H_2C=C$ $CONHR_4$
|
$R_3$

formules dans lesquelles:

— $R_3$ représente l'hydrogène ou un groupe alkyle inférieur en, $C_1$-$C_3$; et

— $R_4$ représente un groupe alkyle ou hydroxyalkyle en $C_1$-$C_{18}$.

Les monomères dans lesquels AA représente KTfa, OrnTfa, KCbz, OrnCbz, Ebzl, EMe, EEt ou Dbzl, c'est-à-dire des amino-acides à chaînes latérales hydrophobes, copolymérisent facilement avec les acrylates et acrylamides en solution organique, tandis que dans le cas des AA tels que Sar, K, Orn, D, E, on peut opérer en solution aqueuse si le co-monomère est soluble. Les polymères qui se présentent généralement sous forme de poudre blanche, sont isolés par précipitation après une éventuelle purification, par exemple, par dialyse, ou des solutions organiques par distillation.

L'étude par diffraction des rayons X, microscopie en lumière polarisée et analyse enthalpique différentielle des polymères ramifiés, tant à l'état sec qu'en solution dans différents solvants, a permis d'établir le caractère à la fois thermotrope et lyotrope de ces composés. L'analyse enthalpique différentielle a permis d'établir le domaine de stabilité des différentes phases liquide-cristallines. La diffraction des rayons X a permis d'établir la structure des différentes phases liquide-cristallines (smectiques de différents types, nématique, cholestérique) et de relier le type de structure des phases liquide-cristallines à la nature des acides aminés AA, à leur degré de polymérisation p et à la longueur du bras espaceur B.

La mise en œuvre de ces composés dans des applications résultant de leur structure de cristaux liquides se fait de façon classique, connue de l'homme de métier.

Les polymères ramifiés préparés à partir d'acides aminés à chaînes latérales hydrophiles tels que Sar, K, Orn, E, D, EnEt, DnEt et pour lesquels p est inférieur à 30 environ, et B a un nombre d'atomes de carbone compris entre 2 et 24 ont aussi des propriétés émulsifiantes et moussantes. Il en est de même des polymères préparés par copolymérisation de lipopeptides polymérisables (I) (obtenus à partir d'amino-acides à chaînes latérales hydrophiles) et d'acrylates ou méthacrylates d'alcoyle, ou d'acryl-ou méthacrylamides ayant de 12 à 18 atomes de carbone dans la chaîne alkyle. Les propriétés émulsifiantes et moussantes de ces polymères ont été testées vis-à-vis de nombreux systèmes eau/hydrocarbures et eau, produits de base de l'industrie cosmétique. Le type et la stabilité des émulsions obtenues ont été étudiés par la méthode des colorants sélectifs, la méthode des dilutions, la conductivité électrique et la microscopie électronique.

Pour obtenir des émulsions, on ajoute aux deux liquides non miscibles de 2 à 10% de polymère en peigne, on agite 10 à 30 minutes et l'émulsion se forme facilement. On a ainsi préparé des émulsions de différentes compositions (de 5 à 60% d'huile) avec les systèmes eau/octane, eau/décane, eau/myristate d'isopropyle, eau/palmitate d'isopropyle, eau/stéarate de butyle ou d'éthyle, eau/cosbiol, eau/mygliol, eau/huile de carnation, eau/huile de vaseline, eau/huile de silicone. Les émulsions obtenues sont très stables (plusieurs mois). On modifie la viscosité, la compacité et les dimensions des émulsions en faisant varier la teneur en polymère ramifié (de 2 à 10%), ou en ajoutant un co-tensioactif (un alcool à longue chaîne, tel que l'alcool cétylique par exemple).

De plus, on peut faire varier la solubilité et la HLB (balance hydrophile-lipophile) des polymères ramifiés en jouant sur la nature de l'acide aminé AA et son degré de polymérisation p, la longueur du bras espaceur B, la nature du squelette polymérique (acrylate, méthacrylate, acrylamide, méthacrylamide) et la composition du polymère ramifié en lipopeptide et en acrylate, méthacrylate, acrylamide ou méthacrylamide à longue chaîne.

Les polymères ramifiés, en particulier sous forme de copolymères de lipopeptides (obtenus à partir d'acides aminés à chaînes latérales hydrophiles) et d'acrylates, méthacrylates acrylamides et méthacrylamides à longues chaînes alkyle (de $C_{12}$ à $C_{18}$) possèdent d'excellentes propriétés émulsifiantes et moussantes et peuvent être utilisées dans l'industrie cosmétique et dermatologique (crèmes hydratantes, crèmes antirides, vernis, shampooings...) ou dans l'industrie pétrolière (lubrifiants, additifs...) etc. Les polymères ramifiés ajoutent à leurs propriétés tensioactives les propriétés des polymères: stabilité, inertie chimique, etc....

La présente invention sera illustrée par les exemples ci-après. Dans les exemples illustrant la préparation des polymères (c'est-à-dire à partir de l'exemple 3), les valeurs de p indiquées sont les degrés moyens de polymérisation en nombre.

*Exemple 1*

Préparation du composé de formule:

$$H_2N\text{-}(CH_2)_n\text{-}Z\text{-}CO\text{-}C(R)=CH_2$$

avec:

- n = 3, 5, 6 ou 12;
- R = H ou $CH_2$; et
- Z = O ou NH.

a) On fait réagir à température ambiante 0,1 mole de carbonate de di-t-butyle $CO(OC_4H_9)_2$ sur 0,1 mole de diamine ou d'aminoalcool [$H_2N(CH_2)_nNH_2$ ou $H_2N(CH_2)_nOH$] en solution dans 300 ml d'un mélange eau/méthanol (50/50 en volume) et en présence d'une quantité suffisante de NaOH pour que le pH soit basique. Après une douzaine d'heures, on précipite le produit final BOC-NH-$(CH_2)_nNH_2$ (ou OH) par addition d'eau et le purifie.

— Pour Z = NH et n = 12, le produit final est séparé du produit de départ par extraction dans le tétrahydrofuranne, puis du dérivé diacylé par chromatographie sur colonne de gel de silice, avec comme éluant du méthanol à 1% de $NH_4OH$.

— Pour Z = NH et n = 6, le dérivé diacylé est insoluble dans l'eau et le produit final est séparé de la diamine de départ par extraction de la solution aqueuse du mélange à l'acétate d'éthyle.

— Pour Z = OH et n = 3 ou 5, le produit final est purifié par chromatographie sur colonne de silice.

b) On introduit goutte à goutte dans une solution de 0,1 mole de ce produit dans 300 ml de THF, 0,15 mole de chlorure d'acryloyle (ou méthacryloyle) en solution dans 200 ml de THF (tétrahydrofuranne),

en présence de 0,15 mole de triéthylamine; le précipité de chlorhydrate de triéthylamine est séparé par filtration et le produit cherché est précipité par addition d'eau, puis le groupe protecteur (BOC) est éliminé par action de l'acide bromhydrique sur le produit en solution dans l'acide acétique ou le THf (0,1 mole de dérivé acylé, dans 125 ml d'acide acétique contenant HBr 4M).

Après quelques heures, on élimine HBr et $CH_3COOH$ par distillation puis lyophilisation et reprend le résidu dans l'eau.

On décompose le sel par addition de NaOH et isole les porduits finaux qui précipitent. On vérifie la pureté des produits obtenus à chacune des étapes par chromatographie en couches minces sur gel de silice, éluant $CH_3OH$ + 1% $NH_4OH$.

Les bandes caractéristiques des spectres infrarouges (KBr) des produits finaux sont pour l'étape a) à 2920, 2840, 1470 (chaîne aliphatique) et 1690, 1175 $cm^{-1}$ (BOC); pour l'étape b) avant élimination du BOC: mêmes bandes que ci-dessus avec 1630 (vinyl), 1660, 1560 (amide) et sans le groupe protecteur: 2920, 2840, 1470 (chaîne aliphatique ) 1630 (vinyle) 1660, 1560 (amide).

*Exemple 2*

Préparation de

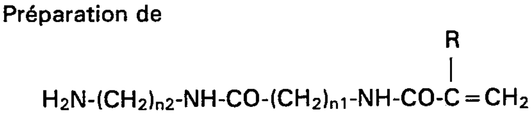

avec:

- n2 = 6 à 12;
- n1 = 4 à 11; et
- R = H ou $CH_3$.

a) acide 12-(acryloylamino)laurique:

$$CH_2=CH-CO-NH-(CH_2)_{11}-COOH$$

L'acide 12-(acrylamino) laurique est obtenu par la méthode décrite par W. De Winter et A. Marien (Makromol. Chem. Rapid. Commun., 5 (1984) 593-96) pour l'acide 11-(méthacryloylamino) undécanoïque.

21,5 g (0,1 mole) d'acide 12-aminolaurique sont dissous dans une solution aqueuse de soude (0,2 mole). On y ajoute goutte à goutte 8,2 ml (0,1 mole) de chlorure d'acryloyle. Après neutralisation à l'acide chlorhydrique, filtration et recristallisation dans l'acétate d'éthyle (en présence d'un peu de 2,6-di-tert-butyl-p-crésol), on obtient 19 g d'acide 12-(acryloylamino) laurique (rendement 70%).

Son spectre IR est caractérisé par les bandes à 1700 $cm^{-1}$ (acide), 1655, 1535 et 3315 $cm^{-1}$ (amide) et 1630 $cm^{-1}$ (double liaison $CH_2=CH$).

On obtient une seule tache en CCM: RF = 0,30 dans $CHCl_3$/MeOH (10/1).

b) 12-(acryloylamino)laurate de succinimidyle:

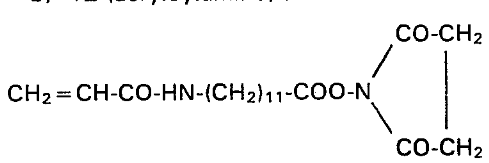

13,45 g (0,05 mole) d'acide 12-acryloylamino) laurique et 6,9 g (0,06 mole) de N-hydroxysuccinimide ainsi qu'un peu de 2,6-di-tert-butyl-p-crésol sont dissous dans 250 ml de THF et la solution est refroidie à 0°C. On y ajoute 10,3 g (0,05 mole) de N,N-dicyclohexylcarbodiimide. Après 20 heures de réaction, le précipité de dicyclohexylurée est filtré, la solution est évaporée, le résidu est repris dans 100 ml d'acétone, la solution est filtrée et évaporée. Le résidu est redissous à chaud dans 300 ml d'éthanol, on laisse refroidir et on ajoute sous agitation un volume d'eau. On filtre, lave et sèche. On récupère 16,5 g d'ester (rendement 90%).

Le spectre IR de l'ester est caractérisée par la disparition de la bande acide à 1700 $cm^{-1}$ et l'existence des bandes à 1660, 1540 et 3330 $cm^{-1}$ (amide), à 1630 $cm^{-1}$ (double liaison acrylique), à 1730, 1745, 1790, 1820, 1210 et 1075 $cm^{-1}$ (ester de succinimidyle).

On obtient une seule tache en CCM: $R_F$ = 0,51 dans $CHCl_3$/MeOH (10/1).

c) N-[12-(acryloylamino) lauryl]1,6-diaminohexane:

$$CH_2=CH-CO-(CH_2)_{11}-CO-NH-(CH_2)_6-NH_2.$$

14,6 g (0,04 mole) de l'ester obtenu en b) sont dissous dans 200 ml d'acétone et ajoutés lentement sous agitation à une solution de 46,4 g (0,4 mole) de 1,6-diaminohexane dans 500 ml de mélange acétone/eau (1/1). On précipite par addition de 2 l d'eau, filtre et lave à l'eau. On reprend le précipité dans 500 ml de mélange acétone/solution aqueuse HCl 0,2 N (1/1 en vol.), on filtre éventuellement et précipite par addition de 2 l de solution de soude 0,05 N. On filtre, lave et sèche. On récupère 12,5 g de produit aminé (rendement 85%).

Le spectre IR du produit est caractérisé par la disparition de toutes les bandes ester de succinimidyle et l'apparition d'une bande amide supplémentaire à 1640 $cm^{-1}$.

On obtient une seule tache en CCM: $R_F$ = 0,3 dans Ethanol 99%, acide acétique 1%, la révélation est faite par la ninhydrine pour l'amine et par la solution aqueuse $NaIO_4$ 1,6%, $KMnO_4$ 0,2% et $Na_2CO_3$ 0,4% pour la double liaison.

Le dosage de l'amine terminale par l'acide perchlorique dans l'acide acétique donne une fonctionnalité de 1.

*Exemple 3*

Fixation du polypeptide

A. *Méthode au N-carboxyanhydride* (NCA)

1 - Préparation du composé de formule:

$$CH_2=CH-CO-NH-(CH_2)_{12}-NH-(Sar)_{6,5}$$

0,06 mole de N-carboxyanhydride de la sarcosine, préparé par action du phosgène sur l'acide aminé, sont mis à réagir avec 0,01 mole de l'amine polymérisable

$$CH_2=CH-CO-N-(CH_2)_{12}\ NH_2$$

obtenue à l'exemple 1 en solution dans 250 ml de tétrahydrofuranne (THF), à température ambiante, sous agitation, pendant 1 heure.

On évapore ensuite le solvant, on lave le résidu par de l'acétone glacée, puis on le dissout dans l'eau avant de lyophiliser.

On détermine le nombre moyen de résidus d'amino acides couplés à l'amine polymérisable en dosant la fonction amine terminale par $HClO_4$ dans $CH_3COOH$. Dans ces conditions, on a mesuré un p moyen = 6,5;
le spectre IR du produit obtenu de formule:

$$CH_2=CHCONH(CH_2)_{12}NH(Sar)_{6,5}$$

est représenté à la figure 1.

2 - Préparation du composé de formule:

$$CH_2=CH\text{-}CO\text{-}NH\text{-}(CH_2)_{12}\text{-}NH\text{-}(Sar)_{30}$$

p prévu = 30; p trouvé = 42, 34 et 21.

0,3 mole de N-carboxyanhydride de sarcosine sont introduits dans une solution de 0,01 mole de l'amine polymérisable:

$$CH_2=CHCONH(CH_2)_{12}NH_2$$

en solution dans 800 ml de THF et 200 ml de méthanol. Après 1 heure d'agitation à température ambiante, on isole le mélange de produits finals comme précédemment.

Le mélange final a été dissous dans le méthanol et par addition d'acétone, on a obtenu avec 81% de rendement par rapport au poids brut trois précipités successifs pesant 1,5 g, 1,3 g et 5 g dont les p étaient respectivement de 42, 34 et 21.

3 - Préparation du composé de formule:

$CH_3$
|
$CH_2=C\text{-}CO\text{-}NH\text{-}(CH_2)_{12}\text{-}NH\text{-}(Sar)_{8,8}$

En faisant réagir seulement 0,1 mole du N-carboxyanhydride de sarcosine sur l'amine

$CH_3$
|
$CH_2=C\text{-}CO\text{-}NH\text{-}(CH_2)_{12}\text{-}NH_2$

en solution dans 200 ml de THF et 50 ml de méthanol, on obtient un lipopeptide de degré de polymérisation, mesuré par dosage de la fonction amine terminale, de p = 8,8.

4 - Préparation du composé de formule:

$$CH_2=CH\text{-}CO\text{-}NH\text{-}(CH_2)_6\text{-}NH\text{-}(Sar)_{5,2}$$

0,05 mole de N-carboxyanhydride de sarcosine sont mis à réagir avec 0,01 mole de l'amine polymérisable

$$CH_2=CH\text{-}CO\text{-}NH\text{-}(CH_2)_6NH_2$$

en solution dans 250 ml de THF, à température ambiante, sous agitation, pendant 1 heure.

Le lipopeptide polymérisable est récupéré par évaporation du solvant, reprise à l'eau et lyophilisation. Le dosage de la fonction amine terminale donne p = 5,2.

5 - Préparation du composé de formule:

$CH_3$
|
$CH_2=C\text{-}COO\text{-}(CH_2)_5\text{-}NH\text{-}(Sar)_{12}$

0,15 mole de N-carboxyanhydride de sarcosine sont mis à réagir avec 0,01 mole de l'amine polymérisable

$CH_3$
|
$CH_2=C\text{-}CO\text{-}O\text{-}(CH_2)_5NH_2$

en solution dans 200 ml de THF et 50 ml de méthanol, à température ambiante, sous agitation, pendant 1 heure. La solution est concentrée et le lipopeptide polymérisable est récupéré par précipitation à l'éther. Le dosage de la fonction amine terminale donne p = 12.

6 - Préparation du composé de formule:

$$CH_2=CH\text{-}CO\text{-}NH\text{-}(CH_2)_{11}\text{-}CO\text{-}NH\text{-}(CH_2)_6\text{-}NH\text{-}(Sar)_6$$

0,07 mole de N-carboxyanhydride de sarcosine sont mis à réagir avec 0,01 mole de l'amine polymérisable

$$CH_2=CH\text{-}CO\text{-}NH\text{-}(CH_2)_{11}\text{-}CO\text{-}NH\text{-}(CH_2)_6NH_2$$

en solution dans 200 ml de THF et 100 ml de méthanol, à température ambiante, sous agitation, pendant 1 heure. On concentre à l'évaporateur rotatif puis précipite le lipopeptide polymérisable à l'éther éthylique. Le dosage de la fonction amine terminale donne p = 6.

7 - Préparation du composé de formule:

$$CH_2=CH\text{-}CO\text{-}NH\text{-}(CH_2)_{12}\text{-}NH\text{-}(KCbz)_{18}$$

0,2 mole de N-carboxyanhydride de carbobenzoxylysine sont mis à réagir avec 0,01 mole de l'amine polymérisable

$$CH_2=CH\text{-}CO\text{-}NH\text{-}(CH_2)_{12}\text{-}NH_2$$

en solution dans 500 ml de THF, à température ambiante, sous agitation, pendant 12 heures. Le lipopeptide polymérisable est précipité dans le méthanol. Le dosage de la fonction amine terminale donne p = 18.

8 - Préparation du composé de formule:

$$CH_2=CH\text{-}CO\text{-}NH\text{-}(CH_2)_{12}\text{-}NH\text{-}(KTfa)_{4,8}$$

0,04 mole de N-carboxyanhydride de trifluoroacétyllysine sont mis à réagir avec 0,01 mole de l'amine polymérisable

$$CH_2=CH\text{-}CO\text{-}NH\text{-}(CH_2)_{12}NH_2$$

en solution dans 200 ml de THF, à température ambiante, sous agitation pendant 6 heures. Le lipopeptide polymérisable est précipité par l'eau.

Le dosage de la fonction amine terminale donne p = 4,8.

9 - Préparation du composé de formule:

$$CH_2=CH\text{-}CO\text{-}NH\text{-}(CH_2)_{12}\text{-}NH\text{-}(K)_{4,8}$$

0,01 mole du lipopeptide

$$CH_2=CH\text{-}CO\text{-}NH\text{-}(CH_2)_{12}NH\text{-}(KTfa)_{4,8}$$

sont dissous dans 250 ml de méthanol puis traités par 250 ml de solution 1N de pipéridine dans l'eau. On laisse réagir 24 heures. On évapore, reprend par un mélange eau/méthanol (90/10 v:v), passe sur une colonne échangeuse d'anions (Dowex 1 × 2) pour éliminer les ions trifluoroacétate, puis lyophilisé. On vérifie par spectroscopie I.R. la disparition des fortes bandes d'absorption du groupe trifluoroacétyle à 1160-1210 $cm^{-1}$.

10 - Préparation du composé de formule:

$$CH_2=CH\text{-}CO\text{-}NH\text{-}(CH_2)_{12}\text{-}NH\text{-}(Ebzl)_{5,2}$$

0,05 mole de N-carboxyanhydride de glutamate de benzyle sont mis à réagir avec 0,01 mole de l'amine polymérisable

$$CH_2=CH\text{-}CO\text{-}NH\text{-}(CH_2)_{12}NH_2$$

en solution dans 500 ml de THF, à température ambiante, sous agitation pendant 12 heures. Le lipopeptide

$$CH_2=CH\text{-}CO\text{-}NH\text{-}(CH_2)_{12}\text{-}NH\text{-}(Ebzl)_p$$

est récupéré par precipitation à l'eau. Le dosage de la fonction amine terminale donne p = 5,2.

11 - Préparation du composé de formule:

$$CH_2=CH\text{-}CO\text{-}NH\text{-}(CH_2)_{12}\text{-}NH\text{-}(E)_{5,2}$$

0,01 mole du lipopeptide

$$CH_2=CH\text{-}CO\text{-}NH\text{-}(CH_2)_{12}\text{-}NH\text{-}(Ebzl)_{5,2}$$

est dissoute dans 100 ml de THF. On traite par la soude en solution alcoolique, puis ajoute progressivement de l'eau jusqu'à obtenir une solution limpide de lipoglutamate de sodium; on concentre et précipite par l'acétone. Le déblocage total de la chaîne latérale de l'acide glutamique est contrôlé par la disparition de l'absorption UV des noyaux benzéniques à 258 nm.

12 - Préparation du composé de formule:

$$CH_2=CH\text{-}CO\text{-}NH\text{-}(CH_2)_{12}\text{-}NH\text{-}(EnEt)_{5,2}$$

0,01 mole du lipopeptide

$$CH_2=CH\text{-}CO\text{-}NH\text{-}(CH_2)_{12}\text{-}NH\text{-}(Ebzl)_{5,2}$$

est dissoute dans 200 ml de dioxane, puis on ajoute 0,1 mole d'aminoéthanol, chauffe à 40°C et laisse réagir sous agitation pendant 8 heures. Le lipopeptide polymérisable

$$CH_2=CH\text{-}CO\text{-}NH\text{-}(CH_2)_{12}\text{-}NH\text{-}(EnEt)_{5,2}$$

est précipité par l'acétone. On contrôle la disparition des groupes benzyle par spectroscopie U.V. à 258 nm.

13 - Préparation du composé de formule:

$$CH_2=CH\text{-}CO\text{-}NH\text{-}(CH_2)_{12}\text{-}NH\text{-}(Dbzl)_{3,8}$$

0,03 mole de N-carboxyanhydride d'aspartate de benzyle sont mises à réagir avec 0,01 mole de l'amine polymérisable

$$CH_2=CH\text{-}CO\text{-}NH\text{-}(CH_2)_{12}\text{-}NH_2$$

en solution dans 500 ml de THF, à température ambiante, sous agitation pendant 12 heures. Le lipopeptide

$$CH_2=CH\text{-}CO\text{-}NH\text{-}(CH_2)_{12}\text{-}NH\text{-}(Dbzl)_p$$

est récupéré par précipitation à l'eau. Le dosage de la fonction amine terminale donne p = 3,8.

14 - Préparation du composé de formule:

$$CH_2=CH\text{-}CO\text{-}NH\text{-}(CH_2)_{12}\text{ -}NH\text{-}(D)_{3,8}$$

0,01 mole du lipopeptide

$$CH_2=CH\text{-}CO\text{-}NH\text{-}(CH_2)_{12}\text{-}NH\text{-}(Dbzl)_{3,8}$$

sont dissoutes dans 100 ml de THF. On traite par la soude en solution alcoolique, puis ajoute progressivement de l'eau jusqu'à obtenir une solution limpide de lipoaspartate de sodium: on concentre et précipite par l'acétone le lipopeptide

$$CH_2=CH\text{-}CO\text{-}NH\text{-}(CH_2)_{12}\text{-}NH\text{-}(D)_{3,8}.$$

On vérifie par spectroscopie U.V. à 258 nm la disparition des groupements benzyle.

15 - Préparation du composé de formule:

$$CH_2=CH\text{-}CO\text{-}NH\text{-}(CH_2)_{12}\text{-}NH\text{-}(KTfa)_{50}$$

p prévu = 50, p trouvé = 70, 40 et 35.

A partir de 0,01 mole de

$$CH_2=CH\text{-}CONH(CH_2)_{12}\ NH_2$$

en solution dans 1 l de THF, on ajoute 0,5 mole de NCA de trifluoroacétyllysine et on laisse sous agitation à température ambiante durant 24 heures. Le produit final, mélange de composés de divers degrés de polymérisation, est précipité par addition d'eau. On fractionne ce mélange par précipitations successives, par addition d'eau à sa solution dans le THF. A partir de 20 g, on obtient 4,1 g de produit avec p = 70; 9,6 g de p = 40 et 2,1 g de p = 35.

16 - Préparation du composé de formule:

$CH_2=CH\text{-}CO\text{-}NH\text{-}(CH_2)_{12}\text{-}NH\text{-}(Ebzl)_{50}$

p prévu = 50; p trouvé = 65, 45 et 36.

A partir de 0,01 mole de

$CH_2=CH\text{-}CO\text{-}NH(CH_2)_{12}NH_2$

en solution dans 1 l de THF et 0,5 mole de NCA de glutamate de benzyle, on obtient un mélange de produits dont le polypeptide terminal a des degrés de polymérisation différents. Ce mélange fractionné dans $THF/H_2O$ donne, avec 75% de rendement, 1,6 g de p = 65; 5,6 g de p = 45 et 1,8 g de p = 36.

B. *Méthode de couplage peptidique*

1 - Préparation de

$CH_2=CH\text{-}CONH(CH_2)_{12}NH\text{-}Sar$

On dissout dans 250 ml de THF, 0,01 mole de

$CH_2=CH\text{-}CONH(CH_2)_{12}\ NH_2$,

0,01 mole de t-butoxycarbonylsarcosine (SarBoc) et 0,01 mole de N-hydroxysuccinimide, puis 0,01 mole de dicyclohexylcarbodiimide. Après 24 heures d'agitation à température ambiante, on élimine la dicyclohexylurée précipitée et précipite le produit obtenu par addition d'un volume d'eau (rendement 95%). Le spectre IR /KBr) du composé présente les bandes intenses: 2920, 2840, 1470 (chaîne aliphatique), 1660, 1555 (amide), 1690, 1175 (BOC), 1630 (vinyle).

On libère ensuite la fonction amine en introduisant 20 ml d'une solution de HCl dans l'éther éthylique (5N) dans le produit précédemment obtenu en solution dans 50 ml de THF, à 0°C. Le chlorhydrate de l'amine précipite lentement, après 24 heures environ à température ambiante. Il est isolé par filtration à 0°C, rendement 92%. le spectre IR (KBr) présente les mêmes bandes, sauf à 1680 et 1175 et a, en plus, une bande à 2420 $cm^{-1}$.

On libère ensuite l'amine de son sel en traitant le chlorhydrate en solution dans 50 ml de méthanol par un équivalent de solution aqueuse de NaOH (1N). Le produit cherché précipite par addition d'un net excès d'eau.

On le purifie ensuite par chromatographie sur colonne de silice en éluant avec du méthanol contenant 1% de $NH_4OH$. Bandes du spectre IR (KBr) $cm^{-1}$: 2920, 2840, 1470, 1660, 1550, 1630.

Chromatographie sur couche mince de gel de silice:

$R_f$ = 0,58 (éluant CH OH + 1% $NH_4OH$).

Pour obtenir le produit de formule:

$CH_2=CH\text{-}CONH(CH_2)_{12}\ NH\text{-}Sar_2$

on fait réagir dans les mêmes conditions la SarBOC sur le produit obtenu précédemment.

2 - Préparation de

$CH_3$
|
$CH_2=C\text{-}CO\text{-}NH\text{-}(CH_2)_{12}\text{-}NH\text{-}Sar$

On dissout dans 250 ml de THF 0,01 mole de

$CH_3$
|
$CH_2=C\text{-}CO\text{-}NH\text{-}(CH_2)_{12}\text{-}NH_2$

0,01 mole de Sar-Boc et 0,01 mole de N-hydroxysuccinimide, puis 0,01 mole de dicyclohexylcarbodiimide. Après 24 heures d'agitation à température ambiante, on élimine le précipité de dicyclohexylurée, puis précipite le produit obtenu par addition d'un volume d'eau (rendement 96%). On libère ensuite la fonction amine de la sarcosine par action de HCl puis NaOH comme dans l'exemple précédent. A chaque étape de la synthèse, les produits sont purifiés et caractérisés comme dans l'exemple précédent.

3 - Préparation de

$CH_2=CH\text{-}CO\text{-}NH\text{-}(CH_2)_{12}\text{-}NH\text{-}Gly$

On dissout dans 250 ml de THF, 0,01 mole de:

$CH_2=CH\text{-}CO\text{-}NH\text{-}(CH_2)_{12}\text{-}NH$,

0,01 mole de t-butyloxycarbonyl-glycine (Gly-Boc) et 0,01 mole de N-hydroxysuccinimide, puis 0,01 mole de dicyclohexylcarbodiimide. Après 24 heures de réaction, sous agitation, à température ambiante, on élimine le précipité de dicyclohexylurée par filtration, puis on précipite le

$CH_2=CH\text{-}CO\text{-}NH\text{-}(CH_2)_{12}\text{-}NH\text{-}Gly\text{-}Boc$

par addition d'un volume d'eau (rendement 94%). On libère ensuite la fonction amine de la glycine par action de HCl puis NaOH comme dans le cas de la sarcosine (exemple 3B1). A chaque étape de la synthèse, les produits obtenus sont purifiés et caractérisés comme à l'exemple 3B1.

*Exemple 4*

Préparation de $(AA)_pNH\text{-}(CH_2)_nNH\text{-}CO\text{-}C=CH_2$
|
R

par l'intermédiaire de $(AA)_pNH\text{-}(CH_2)_nNH_2$

A. $R_1NH\text{-}(CH_2)_n\text{-}NH\text{-}(AA)_p$

avec $R_1$ = groupe protecteur (BOC, Nps...)

1) n = 12, AA = (Sar); p = 12,5

On introduit 0,15 mole de NCA de sarcosine dans 200 ml de THF contenant 0,01 mole de $BOC\text{-}NH\text{-}(CH_2)_{12}NH_2$ et agite à température ambiante pendant 1 heure. Le produit final est isolé par précipitation à l'acétone. Le dosage de la fonction amine terminale donne p = 12,5.

2) n = 12, AA = KTfa, p = 20.

On introduit dans 1 litre de THF contenant 0,01 mole de Boc $NH\text{-}(CH_2)_{12}\text{-}NH_2$, 0,2 mole de NCA de KTfa et on laisse sous agitation, à température ambiante, 24 heures. Le produit final est isolé par précipitation à l'eau.

3) $n = 12$, AA = Ebzl; $p = 25$.

On introduit dans 1 litre de THF contenant 0,01 mole de Nps-NH-$(CH_2)_{12}$-$NH_2$, 0,25 mole de NCA de Ebzl et laisse sous agitation, à température ambiante, 24 heures. Le Nps-NH-$(CH_2)_{12}$ -$NH_2$ est obtenu par action sur la diamine du chlorure de 2-nitrophénylsulfonyle.

Le produit final est isolé par précipitation à l'eau.

B. $CH_2=CR\text{-}CO\text{-}NH\text{-}(CH_2)_n\text{-}NH(AA)_p\text{-}CO\text{-}CH_3$

1) *Blocage du polypeptide:*

$$R_1NH\text{-}(CH_2)_n\text{-}NH\text{-}(AA)_pCOCH_3$$

On dissout 0,1 mole du produit obtenu en A dans 200 ml à 1000 ml de THS selon la valeur de p, avec 0,1 mole de triéthylamine, puis on ajoute 0,15 mole de chlorure d'acétyle et laisse sous agitation à température ambiante, jusqu'à réaction négative à la ninhydrine. On élimine alors le précipité de chlorhydrate de triéthylamine, on évapore les solvants et lyophilise une solution aqueuse du résidu.

2) *Libération de l'amine:*

$$N_2H\text{-}(CH_2)_n\text{-}NH\text{-}(AA)_pCOCH_3.$$

Si $R_1$ = Boc ou Nps, on traite le produit en solution dans le THF par HCl anhydre, puis on libère l'amine de son sel par NaOH méthanolique.

3) *Acylation:*

a) $n = 12$, AA = Sar.

On prépare une solution de 0,01 mole d'amine

$$H_2N\text{-}(CH_2)_{12}\text{-}NH\text{-}(Sar)_{12,5}\text{-}CO\text{-}CH$$

dans 200 ml de THF et 20 ml de methanol. On ajoute lentement et simultanément du chlorure d'acryloyle et son equivalent de triéthylamine (TEA) tous deux en solution dans le THF (quelques ml). On arrête la réaction lorsque la réaction à la ninhydrine devient négative soit pour environ 0,013 mole. La solution est évaporée, le résidu est repris dans l'eau, passé sur une colonne échangeuse d'anions (forme $OH^-$), lyophilisé. Le produit obtenu est repris dans un peu de méthanol et précipité par l'éther diéthylique. On filtre, lave et sèche pour récupérer le lipopeptide

$$CH_2=CH\text{-}CO\text{-}NH\text{-}(CH_2)_{12}\text{-}NH\text{-}(Sar)_{12,5}\text{-}CO\text{-}CH_3.$$

Pour obtenir

$$CH_2=C(CH_3)\text{-}CO\text{-}NH\text{-}(CH_2)_{12}\text{-}NH\text{-}(Sar)_{12,5}\text{-}CO\text{-}CH_3$$

on opère de manière identique, mais remplace le chlorure d'acryloyle par du chlorure de méthacryloyle. Dans les deux cas, le rendement est de 95%.

b) $n = 12$, AA = KTfa.

On prépare une solution de 0,01 mole d'amine

$$H_2N\text{-}(CH_2)_{12}\text{-}NH\text{-}(KTfa)_{20}\text{-}CO\text{-}CH_3$$

dans 200 ml de THF. On ajoute lentement et simultanément du chlorure d'acryloyle et son équivalent de TEA tous deux en solution dans le THF. On arrête la réaction lorsque le test à la ninhydrine devient négatif, soit pour environ 0,015 mole. On filtre le chlorhydrate de TEA puis précipite le lipopeptide

$$CH_2=CH\text{-}CO\text{-}NH\text{-}(CH_2)_{12}\text{-}NH\text{-}(KTfa)_{20}\text{-}CO\text{-}CH_3$$

dans l'eau.

c) $n = 12$; AA = Ebzl

On part de l'amine

$$H_2N\text{-}(CH_2)_{12}\text{-}NH\text{-}(Ebzl)_{25}\text{-}CO\text{-}CH_3$$

on opère dans les mêmes conditions qu'à l'exemple précédent et obtient le lipopeptide

$$CH_2=CH\text{-}CO\text{-}NH\text{-}(Ch_2)_{12}\text{-}NH\text{-}(Ebzl)_{25}\text{-}CO\text{-}CH_3.$$

*Exemple 5:*

Polymérisation.

A. En milieu aqueux.

On laisse, sous agitation, 48 heures, 0,01 mole de:

$$CH_2=\underset{\displaystyle R}{\underset{|}{C}}\text{-}CONH(CH_2)_n\text{-}NH(AA)_p,$$

$5 \times 10^{-4}$ mole de persulfate de potassium et 140 µl de N,N,N',N'-tétraméthyléthylènediamine dans 350 ml d'eau. Le polymère est isolé par lyophilisation, puis purifié par dialyse.

1) On obtient ainsi, à partir du produit isolé à l'exemple 3A1, c'est-à-dire:

$$CH_2=CH\text{-}CONH(CH_2)_{12}\text{-}NH\text{-}(Sar)_{6,5}$$

un composé ayant une viscosité spécifique dans l'eau de 0,25 $dlg^{-1}$ et qui forme une mésophase smectique a température ambiante et se transforme en nématique à 90°C; l'isotropisation a lieu à une température supérieure à 180°C. Ce polymère est aussi lyotrope: à température ambiante, la transition smectique/nématique a lieu pour 23% d'eau, tandis que l'isotropisation se produit pour 50% d'eau environ. Son spectre IR (KBr) est représenté Fig. 2.

2) A partir du lipopeptide

$$CH_2=C\text{-}CH_3\text{-}CO\text{-}NH\text{-}(CH_2)_{12}\text{-}NH\text{-}(Sar)_{12,5}\text{-}CO\text{-}CH_3$$

isolé à l'exemple 4Ba, on obtient un polymère en peigne qui forme une mésophase smectique entre la température ambiante et 120°C; de plus, ce polymère est lyotrope et sa phase smectique existe à la température ambiante pour des teneurs en eau comprises entre 0 et 30%.

B. Polymérisation en solvant organique:

On maintient à 60°C, pendant 48 heures, une solution (du lipopeptide polymérisable et de l'éventuel comonomère) dans le THF, le chloroforme ou un

mélange de THF/choloforme, ayant une concentration de 2 à 5% en poids et contenant $5 \times 10^{-4}$ mole d'azobisisobutyronitrile pour 0,1 mole de monomère. On précipite le polymère ramifié par addition d'un grand volume d'eau et le sèche.

1) Le produit résultant de la polymérisation dans 100 ml d'un mélange THF/chloroforme (50/50 v:v) de 0,01 mole de:

$$CH_2=CH\text{-}CO\text{-}NH(CH_2)_{12}\text{-}NH\text{-}Sar,$$

obtenu à l'exemple 3B1, est smectique à la température ambiante et se transforme en nématique à 60°C environ. Ce polymère est aussi lyotrope: à température ambiante, la transition smectique nématique a lieu pour 35% d'eau, tandis que l'isotropisation se produit pour 50% d'eau environ.

2) Le produit résultant de la copolymérisation dans 200 ml de chloroforme de 0,01 mole de

$$CH_2=CH\text{-}CO\text{-}NH\text{-}(CH_2)_{12}\text{-}NH\text{-}(Sar)_{6,5}$$

et de 0,025 mole de tétradécylacrylamide:

$$CH_2=CH\text{-}CO\text{-}NH\text{-}(CH_2)_{14}\text{-}H$$

donne un copolymère en peigne récupéré par précipitation à l'eau et dont le dosage des fonctions amine par l'acide perchlorique et en solution dans l'acide acétique confirme la composition 1/2,5 en mole. Ce copolymère en peigne est un excellent émulsifiant des produits de base de l'industrie cosmétique.

3) Le produit résultant de la copolymérisation de 0,01 mole de

$$CH_2=CHCONH\text{-}(CH_2)_{12}\text{-}NH(KTfa)_{40}$$

et de 0,04 mole de $N\text{-}(C_{14}H_{28})$acrylamide dans un litre de THF en présence de $5 \times 10^{-4}$ mole d'azobis-isobutyronitrile est nématique à température ambiante tel que ou en solution dans l'acétate d'éthyle ou le dioxane à des concentrations inférieures à 40% environ; dans le méthanol, il est nématique, puis cholestérique. Son spectre IR (film) est représenté Fig. 3. Sa viscosité spécifique, mesurée dans l'acide dichloroacétique est de 0,27 $dlg^{-1}$ à 25°C.

4) 0,01 mole de cpolymère obtenu à l'exemple précédent sont dissoutes dans le méthanol puis traitées par NaOH en solution aqueuse. On évapore, reprend dans l'eau additionnée de 10% de méthanol, passe sur une colonne échangeuse d'anions pour éliminer les ions trifluoroacétate, puis lyophilise. On vérifie par spectroscopie IR la disparition des fortes bandes d'absorption du groupe trifluoroacétyle à 1160-1210 $cm^{-1}$.

5) Le produit résultant de la copolymérisation de 0,01 mole de

$$CH_2=CH\text{-}CO\text{-}NH\text{-}(CH_2)_{12}\text{-}NH\text{-}(Ebzl)_{45}$$

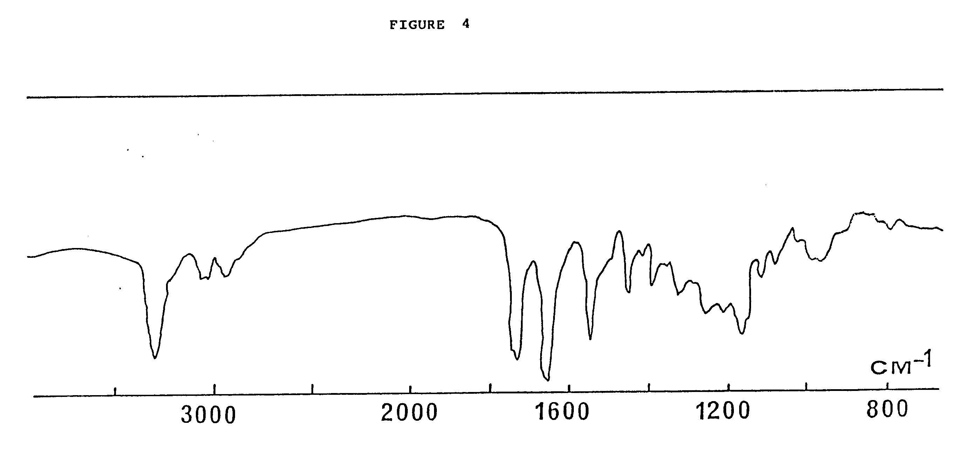

et de 0,05 mole de N-tétradecylacrylamide dans 1 l de THF en présence de $5 \times 10^{-4}$ mole d'AIBN, a une viscosité spécifique dans l'acide dichloroacétique de 0,30 $dlg^{-1}$ à 25°C. Il est nématique à sec et dans le dioxane. Son spectre IR (film) est représenté Fig. 4.

6) 0,01 mole du copolymère obtenu à l'exemple précédent sont dissoutes dans le THF. On traite par une solution alcoolique de soude, puis ajoute progressivement de l'eau jusqu'à obtenir une solution limpide; on concentre et précipite à l'acétone. Le déblocage total de la chaîne latérale d'acide glutamique est contrôlé par la disparition de l'absorption UV des noyaux benzéniques à 258 nm.

7) 0,01 mole de

$$CH_2=CH\text{-}CO\text{-}NH\text{-}(CH_2)_{12}\text{-}NH\text{-}(KCbz)_{18}$$

et 0,03 mole de N-tétradécylacrylamide sont copolymérisés à 60°C en solution dans 1 l de THF par $5 \times 10^{-4}$ mole d'AIBN. Le copolymère obtenu est précipité par le méthanol, dissous dans l'acide acétique et lyophilisé. A température ambiante, il est nématique à l'état sec et en solution dans le dioxane pour des teneurs en solvant inférieures à 40% environ.

8) Le copolymère obtenu à l'exemple précédent est dissous dans l'acide acétique puis traité par HBr, 3M dans l'acide acétique. Le précipité de copolymère (sous forme de bromohydrate) est filtré, lavé à l'acétone et séché. On vérifie par spectroscopie UV l'absence d'absorption due aux noyaux benzéniques à 258 nm.

De la même façon, on a réalisé d'autres polymérisations dont les résultats sont consignés dans les Tableaux II et III ci-après.

TABLEAU II — Autres exemples de polymérisation des lipopeptides

$$CH_2=CR\text{-}CO\text{-}Z\text{-}(CH_2)_n\text{-}NH\text{-}(AA)_p$$

| AA | R | Z | n | p | Solvant | Amorceur |
|---|---|---|---|---|---|---|
| Sar | $CH_3$ | NH | 12 | 1 | $CHCl_3$/THF | AIBN |
| | $CH_3$ | NH | 12 | 8,8 | eau | Persulfate |
| | H | NH | 12 | 12,5 | eau | Persulfate |
| | H | NH | 12 | 21 | eau | Persulfate |
| | H | NH | 12 | 34 | eau | Persulfate |
| | H | NH | 12 | 42 | eau | Persulfate |
| | H | NH | 6 | 5,2 | eau | Persulfate |
| | $CH_3$ | O | 5 | 12 | eau | Persulfate |

TABLEAU II (suite) — Autres exemples de polymérisation des lipopeptides

$CH_2=CR\text{-}CO\text{-}Z\text{-}(CH_2)_n\text{-}NH\text{-}(AA)_p$

| AA | R | Z | n | p | Solvant | Amorceur |
|---|---|---|---|---|---|---|
| K | H | NH | 12 | 4,8 | THF | AIBN |
| E | H | NH | 12 | 5,2 | eau | Persulfate |
| EnEt | H | NH | 12 | 5,2 | THF | AIBN |
| D | H | NH | 12 | 3,8 | eau | Persulfate |

Ces homopolymères se sont révélés présenter des propriétés de cristaux liquides.

TABLEAU III — Autres exemples de copolymérisation de

$CH_2=CR\text{-}CO\text{-}Z\text{-}(CH_2)_n\text{-}NH\text{-}(AA)_p$ (I)
avec $CH_2=CR'\text{-}CO\text{-}Z'\text{-}(CH_2)_n\text{-}H$ (II)

| AA | R | Z | n | p | R' | Z' | n' | I/II en moles |
|---|---|---|---|---|---|---|---|---|
| Sar | H | NH | 12 | 6,5 | H | NH | 14 | 1/1,6 |
| | $CH_3$ | NH | 12 | 8,8 | $CH_3$ | NH | 18 | 1/2 |
| | $CH_3$ | NH | 12 | 8,8 | $CH_3$ | NH | 18 | 1/3 |
| | $CH_3$ | NH | 12 | 8,8 | $CH_3$ | NH | 18 | 2/1 |
| | $CH_3$ | O | 5 | 12 | $CH_3$ | O | 16 | 1/3 |
| K | H | NH | 12 | 4,8 | H | NH | 18 | 1/3 |
| E | H | NH | 12 | 5,2 | H | NH | 12 | 1/3 |
| EnEt | H | NH | 12 | 5,2 | H | NH | 16 | 1/2 |

Toutes les copolymérisations sont réalisées en solution dans le THF en utilisant l'AIBN comme amorceur.

Ces copolymères se sont révélés présenter de bonnes propriétés émulsionnantes et moussantes.

## Revendications

1. Polymères résultant de l'homopolymérisation ou de la copolymérisation de monomères de formule I:

$H_2C=C\text{-}C\text{-}Z\text{-}B\text{-}NH\text{-}(AA)_p$ (I)
| ||
R O

formule dans laquelle:

— R est H ou un groupe alkyle inférieur en $C_1$ à $C_3$;

— Z est O ou NH;

— B répond à la formule:

$$-(CH_2)_{q1}\text{-}Z_1\text{-}(CH_2)_{q2}\text{-}$$

formule dans laquelle $q_1$ et $q_2$ sont des entiers supérieurs ou égaux à 1, dont la somme est inférieure à 32 et $Z_1$ représente une simple liaison ou O, S, NH, CO ou CO-NH.

— $(AA)_p$ représente un radical peptidique constitué de p alpha-aminoacides aliphatiques couplés, et fixé à B par sa fonction carboxyle, chaque aminoacide comportant moins de 15 atomes de carbone et étant éventuellement substitué sur un carbone de la chaîne par un groupe acide ou ester cabroxylique, amide ou amine, la fonction amine terminale du radical peptidique pouvant être acylée par un groupe R'CO, R' étant un radical alkyle renfermant de 1 à 4 atomes de carbone, ou par tout autre groupe stable dans les conditions de la synthèse; et

— p représentant un nombre compris entre 1 et 200.

2. Polymères selon la revendication 1, caractérisés en ce que B comporte 6 à 24 atomes de carbone.

3. Polymères selon l'une des revendications 1 ou 2, caractérisés en ce que AA est dérivé d'acides aminés possédant une chaîne latérale hydrophile.

4. Polymères selon la revendication 3, caractérisés en ce que AA est le dérivé de la sarcosine, de l'hydroxyéthylglutamine et de l'hydroxyéthylaspartamine.

5. Polymères selon la revendication 4, caractérisés par le fait que AA est le dérivé de la sarcosine.

6. Polymères selon l'une des revendications 1 à 5,

caractérisé en ce qu'ils résultant de la copolymérisation d'un monomère de formule I décrit à la revendication 1, avec un acrylate d'alkyle ou d'hydroxyalkyle de formule:

$$CH_2=\overset{R_3}{\overset{|}{C}}\text{-}COOR_4$$

ou un acrylamide de formule:

$$CH_2=\underset{R}{\underset{|}{C}}\text{-}CONHR_4$$

formules dans lesquelles:

— $R_3$ est H ou un groupe alkyle inférieur en $C_1$-$C_3$; et

— $R_4$ représente un groupe alkyle ou hydroxyalkyle en $C_1$ à, $C_{18}$.

7. Polymères selon la revendication 6, caractérisés en ce que AA représente un dérivé d'acide aminé à chaîne latérale hydrophobe, choisi parmi la trifluoroacétyllysine, la carbobenzoxylysine, la trifluoroacétylornithine les glutamates d'alkyle et le glutamate de benzyle.

8. Polymères selon la revendication 6, caractérisés par le fait que AA représente un dérivé d'acide aminé possédant une chaîne latérale hydrophile et choisi parmi la sarcosine, la lysine, l'ornithine, l'acide glutamique, l'acide aspartique, l'hydroxyéthylglutamine et l'hydroxyéthylaspartamine.

9. Polymères selon la revendication 6, caractérisés par le fait que AA représente un dérivé d'acide amine possédant une chaîne latérale hydrophile et constitué par la sarcosine.

10. Polymères selon l'une des revendications 1 à 9, caractérisés en ce qu'ils résultent de la copolymérisation d'au moins 2 monomères de formule (I).

11. Compositions de polymères selon l'une des revendications 1 à 10, caractérisées en ce qu'elles sont des mélanges de polymères de différents degrés de polymérisation, ou de p différents.

12. Monomères de formule (I):

$$H_2C=\underset{R}{\underset{|}{C}}\text{-}\underset{O}{\underset{\|}{C}}\text{-}Z\text{-}B\text{-}NH\text{-}(AA)_p$$

formule dans laquelle

— R est H ou un groupe alkyle inférieur en $C_1$ à $C_3$;

— Z est O ou NH;

— B répond à la formule:

— $(CH_2)_{q1}$-$Z_1$-$(CH_2)_{q2}$-

formule dans laquelle $q_1$ et $q_2$ sont des entiers supérieurs ou égaux à 1, dont la somme est inférieure à 32 et $Z_1$ représente une simple liaison ou O, S, NH, CO ou CO-NH.

— $(AA)_p$ représente un radical peptidique constitué de p alpha-aminoacides aliphatiques couplés, et fixé à B par sa fonction carboxyle, chaque aminoacide comportant moins de 15 atomes de carbone et étant éventuellement substitué sur un carbone de la chaîne par un groupe acide ou ester carboxylique, amide ou amine, la fonction amine terminale du radical peptidique pouvant être acylée par un groupe R'CO, R' étant un radical alkyle renfermant de 1 à 4 atomes de carbone, ou par tout autre groupe stable dans les conditions de la synthèse; et

— p représentant un nombre compris entre 1 et 200.

13. Cristaux liquides thermotropes et lyotropes constitués de polymères selon l'une quelconque des revendications 1 à 11.

14. Agents émulsionnants et moussants constitués d'homopolymères selon la revendication 1 et tels que dans la formule (I):

$$H_2C=\underset{R}{\underset{|}{C}}\text{-}\underset{O}{\underset{\|}{C}}\text{-}Z\text{-}B\text{-}NH\text{-}(AA)_p$$

— R soit H ou un groupe alkyle inférieur;

— Z soit O ou NH;

— B représente une chaîne alkyle en $C_2$ à $C_{24}$;

— AA soit dérivé d'un acide aminé à chaîne latérale hydrophile; et

— p soit inférieur à 30.

15. Agents émulsionnants et moussants constitués de copolymères selon la revendication 6 et tels que dans la formule (I) donnée à la revendication 1, R soit H ou un groupe alkyle inférieur, Z soit O ou NH, B représente une chaîne alkyle en $C_2$ à $C_{24}$, AA soit dérivé d'un acide amine à chaîne latérale hydrophile et p soit inférieur à 30.

16. Agents emulsionnants et moussants selon l'une des revendications 14 et 15, caractérisés par le fait que AA est le dérivé de la sarcosine.

17. Procédé de préparation de composés selon la revendication 12, caractérisé en ce que:

a) on fait réagir un dérivé activé de:

$$H_2C=\overset{R}{\overset{|}{C}}\text{-}COOH$$

sur $HZ$-$B$-$NH_2$;

b) on fait réagir sur:

$$H_2C=\overset{R}{\overset{|}{C}}\text{-}CO\text{-}Z\text{-}B\text{-}NH_2$$

obtenu, le N carboxyanhydride de l'aminoacide ou, dans les conditions d'un couplage peptidique, l'aminoacide dont l'amine est protégée, avec les groupes secondaires de l'aminoacide pouvant réagir, protégés;

c) on libre les fonctions protégées.

18. Procédé de préparation de composés selon la revendication 12, caractérisé en ce que l'on fait réagir un dérivé activé de:

R
|
$H_2C=C\text{-}COOH$

sur un composé de formule $HZ\text{-}B\text{-}NH\text{-}(AA)_p$ dont la fonction amine libre du groupe peptidique est bloquée.

19. Procédé de préparation de composés selon la revendication 12, caractérisé en ce que:

a) on fait réagir un dérivé activé de

R
|
$H_2C=C\text{-}COOH$

sur $H_2N\text{-}(CH_2)_{n1}\text{-}COOH$

avec n1 compris entre 2 et 11.

b) on active le dérivé

R
|
$H_2C=C\text{-}CO\text{-}NH\text{-}(CH_2)_{n1}\text{-}COOH$

obtenu, puis le fait réagir avec une diamine

$H_2N\text{-}(CH_2)_{n2}\text{-}NH_2$ avec

n2 = entier de 2 à 20.

c) on fait réagir sur

R
|
$H_2C=C\text{-}CO\text{-}NH\text{-}(CH_2)_{n1}\text{-}CO\text{-}NH\text{-}(CH_2)_{n2}\text{-}NH_2$

obtenu, le N-carboxyanhydride de l'amino-acide ou, dans les conditions d'un couplage peptidique, l'amino-acide dont l'amine est protégée, avec les groupes secondaires de l'amino-acide pouvant réagir, protégés.

d) on libère les fonctions protégées.

**Patentansprüche**

1. Polymere, hervorgehend aus einer Homopolymerisation oder einer Copolymerisation von Monomeren der Formel (I):

$H_2C=C\text{-}C\text{-}Z\text{-}B\text{-}NH\text{-}(AA)_p$ (I)
| ||
R O

worin

— R H oder eine niedrige $C_1$ bis $C_3$ Alkylgruppe ist;

— Z O oder NH ist;

— B der Formel

$-(CH_2)_{q1}\text{-}Z_1\text{-}(CH_2)_{q2}-$

entspricht, worin $q_1$ und $q_2$ ganze Zahlen größer oder gleich 1 sind, deren Summe kleiner als 32 ist und

— Z eine einfache Bindung oder O, S, NH, CO oder CO-NH bedeutet;

— $(AA)_p$ einen peptidischen Rest bedeutet, der aus p aliphatisch verknüpfen α-Aminosäuren besteht und der über seine Carboxylfunktion mit B verknüpft ist und wobei jede Aminosäure weniger als 15 C-Atome enthält und wobei gegebenenfalls ein Kohlenstoffatom der Kette durch eine Säure- oder Carboxylestergruppe, ein Amid oder ein Amin substituiert ist; die endständige Aminfunktion des peptidischen Restes durch eine Gruppe R'CO, in der R' ein Alkyrest mit 1 bis 4 C-Atomen ist, oder durch irgendeine andere Gruppe, die unter den Bedingungen der Synthese stabil ist, acyliert sein kann; und

— p eine Zahl zwischen 1 und 200 bedeutet.

2. Polymere nach Anspruch 1, dadurch gekennzeichnet, daß B 6 bis 24 C-Atome enthält.

3. Polymere nach einem der Ansprüche 1 oder 2, dadurch gekennzeichnet, daß AA von Aminosäuren abgeleitet ist, die eine hydrophile Seitenkette besitzen.

4. Polymere nach Anspruch 3, dadurch gekennzeichnet, daß AA das Derivat von Sarkosin, von Hydroxyethylglutamin und von Hydroxyethylaspartamin ist.

5. Polymere nach Anspruch 4, dadurch gekennzeichnet, daß AA das Derivat von Sarkosin ist.

6. Polymere nach einem der Ansprüche 1 bis 5, dadurch gekennzeichnet, daß sie aus einer Copolymerisation eines Monomers nach Formel (I), beschrieben in Anspruch 1, mit einem Alkylacrylat oder einem Hydroxyalkylacrylat der Formel:

$R_3$
|
$CH_2=C\text{-}COOR_4$

oder einem Acrylamid der Formel:

$CH_2=C\text{-}CONHR_4$
|
R

hervorgehen, worin

— $R_3$ H oder eine niedrige $C_1$ bis $C_3$-Alkylgruppe ist; und

— $R_4$ eine $C_1$ bis $C_{18}$-Alkylgruppe oder $C_1$ bis $C_{18}$-Hydroxyalkylgruppe bedeutet.

7. Polymere nach Anspruch 6, dadurch gekennzeichnet, daß AA ein Derivat eines sauren Amins mit hydrophober Seitenkette ist, das ausgewählt ist aus Trifluoroacetyllysin, Carbobenzoxylysin, Trifluoroacetylornithin, Alkylglutamaten und Benzylglutamat.

8. Polymere nach Anspruch 6, dadurch gekennzeichnet, daß AA ein Derivat eines sauren Amins ist, das eine hydrophile Seitenkette besitzt und ausgewählt ist aus Sarkosin, Lysin, Ornithin, Glutaminsäure, Asparaginsäure, Hydroxyethylglutamin und Hydroxyethylaspartamin.

9. Polymere nach Anspruch 6, dadurch gekennzeichnet, daß AA ein saures Amin ist, das eine hydrophile Seitenkette besitzt und aus Sarkosin besteht.

10. Polymere nach einem der Ansprüche 1 bis 9, dadurch gekennzeichnet, daß sie aus der Copolymerisation von wenigstens zwei Monomeren der Formel (I) hervorgehen.

11. Zusammensetzungen von Polymeren nach einem der Ansprüche 1 bis 10, dadurch gekennzeichnet, daß sie Mischungen von Polymeren mit verschiedenen Polymerisationsgraden oder verschiedenen p-Werten sind.

12. Monomere der Formel (I):

$$H_2C=C-C-Z-B-NH-(AA)_p \quad (I)$$
$$\;\;\;\;| \;\;\|$$
$$\;\;\;\;R \;\;O$$

worin

— R H oder eine niedrige $C_1$ bis $C_3$ Alkylgruppe ist;

— Z O oder NH ist;

— B der Formel

$$-(CH_2)_{q1}-Z_1-(CH_2)_{q2}-$$

entspricht, worin $q_1$ und $q_2$ ganze Zahlen größer oder gleich 1 sind, deren Summe kleiner als 32 ist und

— Z eine einfache Bindung oder O, S, NH, CO oder CO-NH bedeutet;

— $(AA)_p$ einen peptidischen Rest bedeutet, der aus p aliphatisch verknüpften α-Aminosäuren besteht und der über seine Carboxylfunktion mit B verknüpft ist und wobei jede Aminosäure weniger als 15 C-Atome enthält und wobei gegebenenfalls ein Kohlenstoffatom der Kette durch eine Säure- oder Carboxylestergruppe, ein Amid oder ein Amin substituiert ist; die endständige Aminfunktion des peptidischen Restes durch eine Gruppe R'CO, in der R' ein Alkyrest mit 1 bis 4 C-Atomen ist, oder durch irgendeine andere Gruppe, die unter den Bedingungen der Synthese stabil ist, acyliert sein kann; und

— p eine Zahl zwischen 1 und 299 bedeutet.

13. Thermotrope und lyotrope Flüssigkristalle, die aus den Polymeren nach einem der Ansprüche 1 bis 11 bestehen.

14. Emulgier- und Schaummittel, die aus Homopoylmeren nach Anspruch 1 entsprechend der Formel (I) aufgebaut sind:

$$H_2C=C-C-Z-B-NH-(AA)_p \quad (I)$$
$$\;\;\;\;| \;\;\|$$
$$\;\;\;\;R \;\;O$$

— R ist H oder eine niedrige Alkylgruppe;

— Z ist O oder NH;

— B ist eine $C_2$ bis $C_{24}$ -Alkylkette;

— AA ist von einem sauren Amin mit einer hydrophilen Seitenkette abgeleitet; und

— p ist eine Zahl unter 30.

15. Emulgier- und Schaummittel, die aus einem Copolymer nach Anspruch 6 und entsprechend der Formel (I) nach Anspruch 1 aufgebaut sind, worin R H oder eine niedrige Alkylgruppe ist, Z O oder NH ist, B eine $C_2$ bis $C_{24}$-Alkylkette ist, AA von einem sauren Amin mit einer hydrophilen Seitenkette abgeleitet ist und p eine Zahl unter 30 ist.

16. Emulgier- und Schaummittel nach einem der Anprüche 14 und 15, dadurch gekennzeichnet, daß AA das Derivat von Sarkosin ist.

17. Verfahren zur Herstellung der Verbindungen nach Anspruch 12, dadurch gekennzeichnet, daß man

a) ein aktives Derivat der Formel:

$$\;\;\;\;R$$
$$\;\;\;\;|$$
$$H_2C=C-COOH$$

mit $HZ-B-NH_2$ reagieren läßt;

b) das arhaltene:

$$\;\;\;\;R$$
$$\;\;\;\;|$$
$$H_2C=C-CO-Z-B-NH_2$$

mit dem N-Carboxyanhydrid der Aminosäure oder unter den Bedingungen einer Peptidkopplung mit einer Aminosäure, deren Amin geschützt ist und wobei die sekundären Gruppen der Aminosäure, die reagieren können, geschützt sind, umsetzt;

c) die Schutzgruppen entfernt.

18. Verfahren zur Herstellung der Verbindungen nach Anspruch 12, dadurch gekennzeichnet, daß man ein aktives Derivat der Formel:

$$\;\;\;\;R$$
$$\;\;\;\;|$$
$$H_2C=C-COOH$$

mit einer Verbindung der Formel $HZ-B-NH-(AA)_p$, deren freie Aminfunktion der Peptidgruppe blockiert ist, reagieren läßt.

19. Verfahren zur Herstellung von Verbindungen nach Anspruch 12, dadurch gekennzeichnet, daß man:

a) ein Derivat der Formel

$$\;\;\;\;R$$
$$\;\;\;\;|$$
$$H_2C=C-COOH$$

mit $H_2N-(CH_2)_{n1}-COOH$, worin n1 zwischen 2 und 11 liegt, umsetzt;

b) das erhaltene Derivat

$$\;\;\;\;R$$
$$\;\;\;\;|$$
$$H_2C=C-CO-NH-(CH_2)_{n1}-COOH$$

aktiviert und anschließend mit einem Diamin

$$H_2N-(CH_2)_{n2}-NH_2,$$

worin n2 eine ganze Zahl zwischen 2 und 20 ist, umsetzt;

c) das erhaltene

$$\;\;\;\;R$$
$$\;\;\;\;|$$
$$H_2C=C-CO-NH-(CH_2)_{n1}-CO-NH-(CH_2)_{n2}-NH_2$$

mit dem N-Carboxyanhydrid einer Aminosäure oder unter den Bedingungen einer Peptidkupplung mit einer Aminosäure, deren Amin geschützt ist, und wobei die sekundären Gruppen der Aminosäure, die reagieren können, geschützt sind, umsetzt;

d) die Schutzgruppen entfernt.

**Claims**

1. Polymers resulting from the homopolymerization or the copolymerization of monomers of formula I:

$$H_2C=\underset{R}{\underset{|}{C}}-\underset{O}{\underset{\|}{C}}-Z-B-NH-(AA)_p \qquad (I)$$

in which formula:
— R is H or a $C_1$ to $C_3$, lower alkyl group;
— Z is O or NH;
— B corresponds to the formula:

$$-CH_2)_{q1}-Z_1-(CH_2)_{q2}-$$

in which formula $q_1$ and $q_2$ are integers greater than or equal to 1, whose sum is less than 32, and $Z_1$ represents a single bond or O, S, NH, CO or CO-NH;
— $(AA)_p$ represents a peptide radical constituted by p linked aliphatic alpha-amino acids and joined to B by its carboxyl group, each amino acid comprising less than 15 carbon atoms and optionally being substituted on one carbon in the chain by a carboxylic acid or ester group or amide or amine group, the terminal amine group of the peptide radical being able to be acylated by a R'CO group, R' being an alkyl radical containing 1 to 4 carbon atoms, or by any other group which is stable under the conditions of the synthesis; and
— p representing a number between 1 and 200.

2. Polymers according to Claim 1, characterized in that B comprises 6 to 24 carbon atoms.
3. Polymers according to one of Claims 1 or 2, characterized in that AA is derived from amino acids possessing a hydrophilic side chain.
4. Polymers according to Claim 3, characterized in that AA is derived from sarcosine, hydroxyethylglutamine and hydroxyethylaspartamine.
5. Polymers according to Claim 4 characterized by the fact that AA is derived from sarcosine.
6. Polymers according to one of Claims 1 to 5, characterized in that they result from the copolymerization of a monomer of formula I described in Claim 1, with an alkyl or hydroxyalkyl acrylate of formula:

$$CH_2=\overset{R_3}{\overset{|}{C}}-COOR_4$$

or an acrylamide of formula:

$$CH_2=\underset{R}{\underset{|}{C}}-CONHR_4$$

in which formulae:
— $R_3$ is H or a $C_1$-$C_3$ lower alkyl group; and
— $R_4$ represents a $C_1$ to $C_{18}$ alkyl or hydroxyalkyl group.

7. Polymers according to Claim 6, characterized in that AA represents a derivative of an amino acid with a hydrophobic side chain, chosen from trifluoroacetyllysine, carbobenzoxylysine, trifluoroacetylornithine, the alkyl glutamates and benzyl glutamate.
8. Polymers according to Claim 6, characterised by the fact that AA represents an amino acid derivative possessing a hydrophilic side chain and chosen from sarcosine, lysine, ornithine, glutamic acid, aspartic acid, hydroxymethylglutamine and hydroxyethylaspartamine.
9. Polymers according to Claim 6, characterized by the fact that AA represents an amino acid derivative possessing a hydrophilic side chain and constituted by sarcosine.
10. Polymers according to one of Claims 1 to 9, characterized in that they result from the copolymerization of at least 2 monomers of formula (I).
11. Compositions of polymers according to one of Claims 1 to 10, characterized in that they are mixtures of polymers with different degrees of polymerization, or with different p.
12. Monomers of formula (I):

$$H_2C=\underset{R}{\underset{|}{C}}-\underset{O}{\underset{\|}{C}}-Z-B-NH-(AA)_p \qquad (I)$$

in which formula:
— R is H or a $C_1$ to $C_3$ lower alkyl group;
— Z is O or NH;
— B corresponds to the formula:

$$-(CH_2)_{q1}-Z_1-(CH_2)_{q2}-$$

in which formula $q_1$ and $q_2$ are integers greater than or equal to 1, whose sum is less than 32, and $Z_1$ represents a single bond or O, S, MH, CO or CO-NH;
— $(AA)_p$ represents a peptide radical constituted by p linked aliphatic alpha-amino acids and joined to B by its carboxyl group, each amino acid comprising less than 15 carbon atoms and optionally being substituted on one carbon in the chain by a carboxylic acid or ester group or amide or amine group, the terminal amine group of the peptide radical being able to be acylated by a R'CO group, R' being an alkyl radical containing 1 to 4 carbon atoms, or by any other group which is stable under the conditions of the synthesis; and
— p representing a number between 1 and 200.

13. Thermotropic and lyotropic liquid crystals constituted of polymers according to any one of Claims 1 to 11.

14. Emulsifying and foaming agents constituted of homopolymers according to Claim 1 and such that in the formula (I):

$$H_2C=C\text{-}C\text{-}Z\text{-}B\text{-}NH\text{-}(AA)_p \quad (I)$$
| ||
R O

— R is H or a lower alkyl group;
— Z is O or NH;
— B represents a $C_2$ to $C_{24}$ alkyl chain;
— AA is derived from an amino acid with a hydrophilic side chain; and
— p is less than 30.

15. Emulsifying and foaming agents constituted of polymers according to Claim 6 and such that in the formula (I) given in Claim 1, R is H or a lower alkyl group, Z is O or NH, B represents a $C_2$ to $C_{24}$ alkyl chain, AA is derived from an amino acid with a hydrophilic side chain and p is less than 30.

16. Emulsifying and foaming agents according to one of Claims 14 and 15, characterized by the fact that AA is derived from sarcosine.

17. Process for the preparation of compounds according to Claim 12, characterized in that:

a) an activated derivative of:

R
|
$H_2C=C\text{-}COOH$

is reacted with $HZ\text{-}B\text{-}NH_2$;

b) the

R
|
$H_2C=C\text{-}CO\text{-}Z\text{-}B\text{-}NH_2$

obtained is reacted with the N-carboxyanhydride of the amino acid or, under the conditions of a peptide linkage, with the amino acid whose amine is protected, with the secondary groups of the amino acid which are capable of reacting being protected;

c) the protected groups are liberated.

18. Process for the preparation of compounds according to Claim 12, characterized in that an activated derivative of:

R
|
$H_2C=C\text{-}COOH$

is reacted with a compound of formula

$HZ\text{-}B\text{-}NH\text{-}(AA)_p$

in which the free amine group of the peptide group is blocked.

19. Process for the preparation of compounds according to Claim 12, characterized in that:

a) an activated derivative of

R
|
$H_2C=C\text{-}COOH$

is reacted with $H_2N\text{-}(CH_2)_{n1}\text{-}COOH$, where n1 is between 2 and 11;

b) the derivative

R
|
$H_2C=C\text{-}CO\text{-}NH\text{-}(CH_2)_{n1}\text{-}COOH$

obtained is activated and then reacted with a diamine $H_2N\text{-}(CH_2)_{n2}\text{-}NH_2$ where n2 is an integer from 2 to 20;

c) the

R
|
$H_2C=C\text{-}CO\text{-}NH\text{-}(CH_2)_{n1}\text{-}CO\text{-}NH\text{-}(CH_2)_{n2}\text{-}NH_2$

obtained is reacted with the N-carboxyanhydride of the amino acid or, under the conditions of a peptide linkage, with the amino acid whose amine is protected, with the secondary groups of the amino acid which are capable of reacting being protected.

FIGURE 1

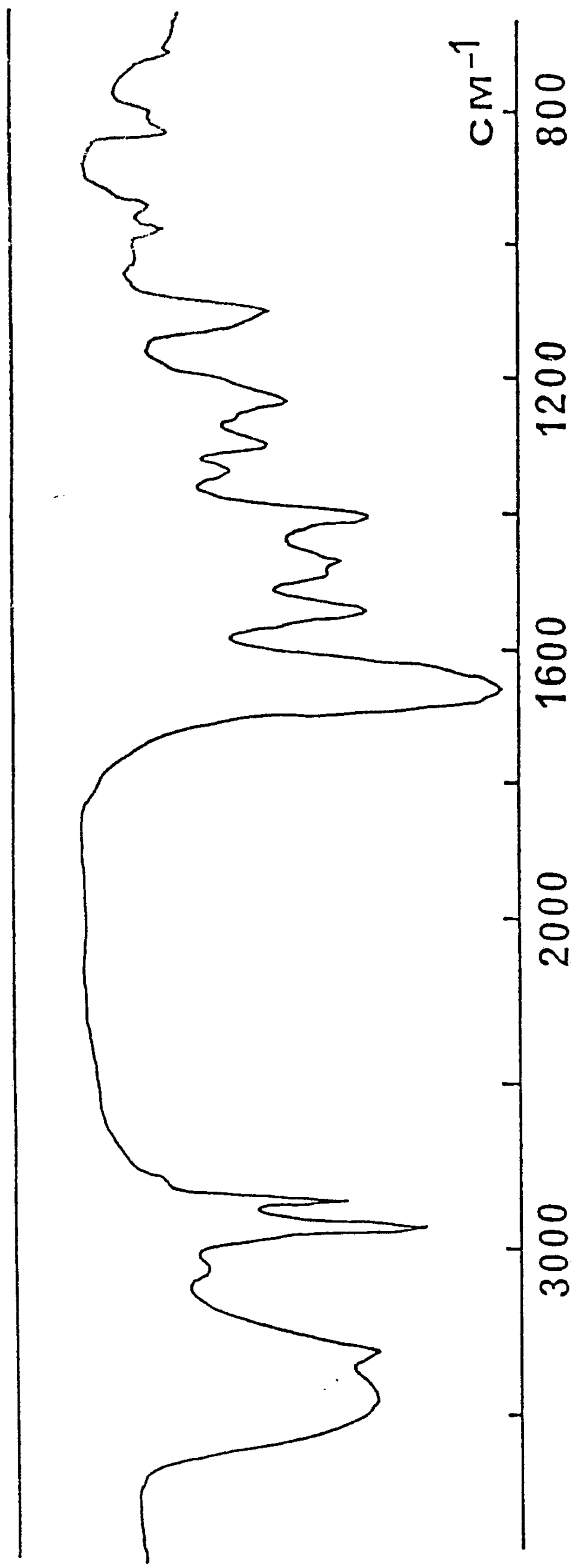

FIGURE 2

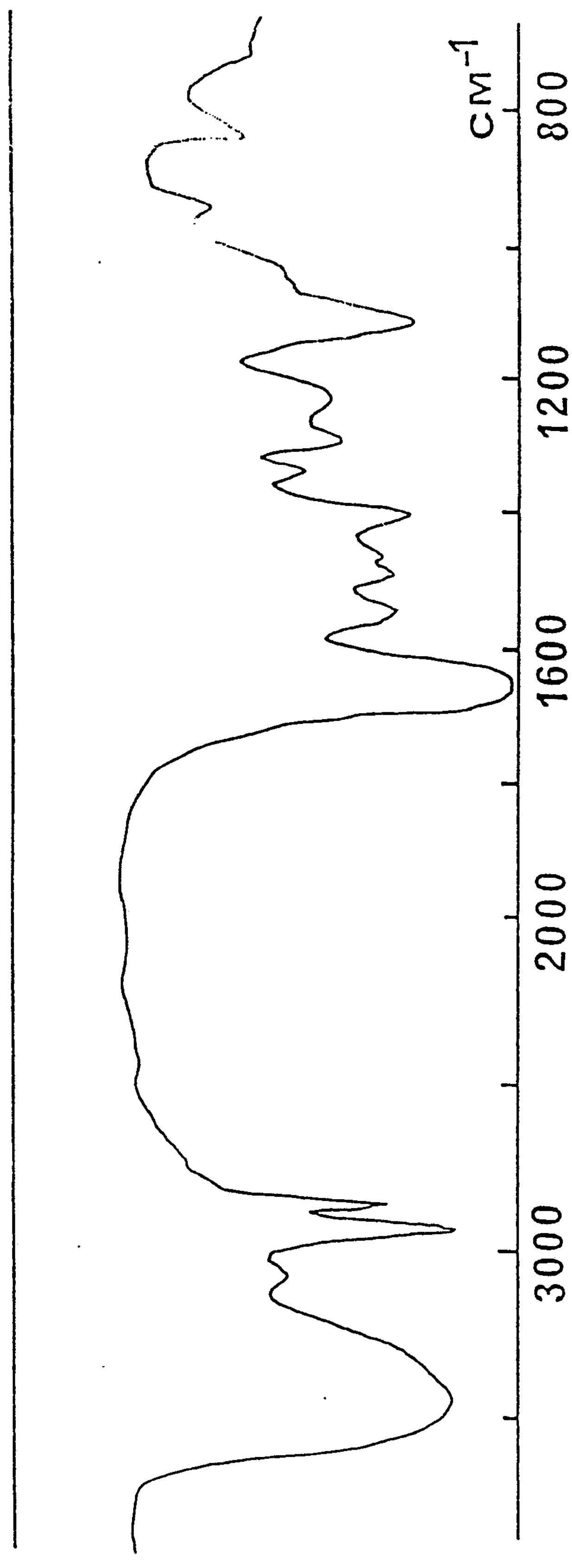

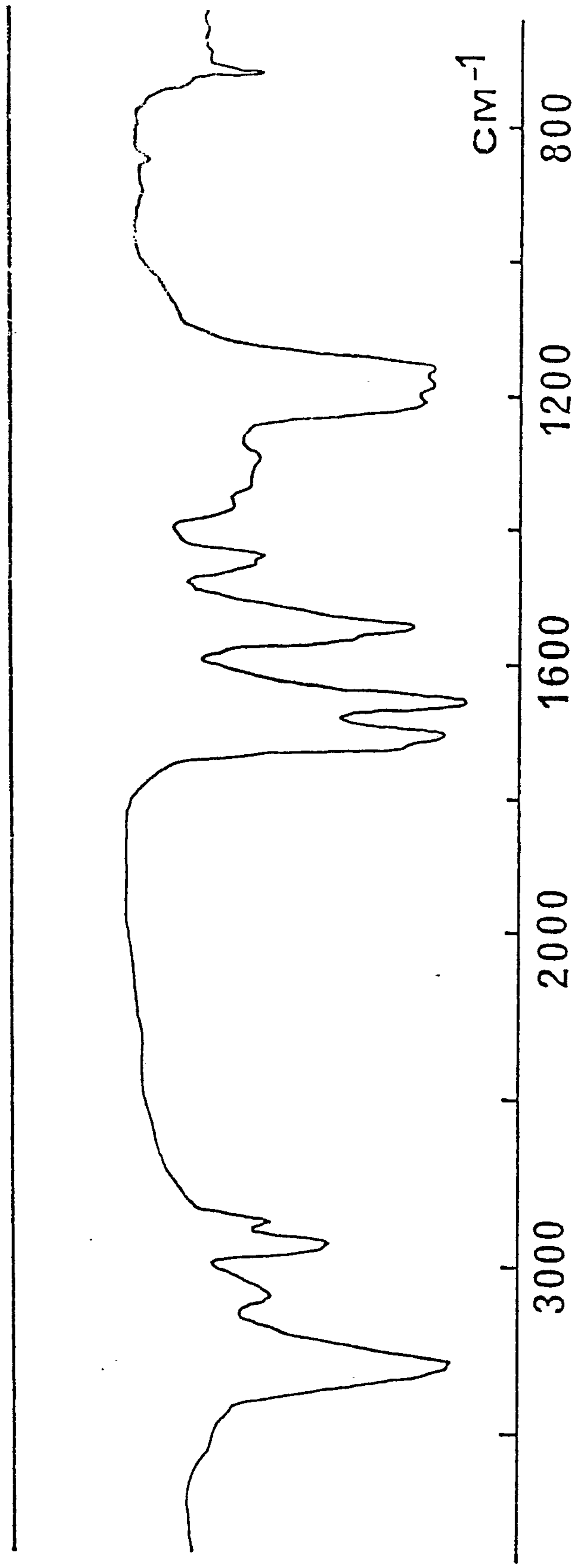


figure 3

FIGURE 4